# EUROPEAN PATENT APPLICATION

(11) **EP 4 599 760 A1**
(43) Date of publication of application: **13.08.2025**
(21) Application number: 24175938.0
(22) Date of filing: 15.05.2024
(51) Int. Cl.: A61B 5/03, A61B 5/00

(54) **BONE-CONDUCTION TRANSCRANIAL CEREBRAL PRESSURE MONITORING DEVICE AND BONE-CONDUCTION TRANSCRANIAL CEREBRAL PRESSURE MONITORING MODULE**

(30) Priority: 07.02.2024 TW 113201508 U
(71) Applicant: Flow Ehealth Inc, Taipei City 115601 (TW); Liu, Chia-Shing, Taipei City 106066 (TW)
(72) Inventor: Liu, Chia-Shing, 106066 Taipei City (TW)
(74) Representative: VKK Patentanwälte PartG mbB

(57) **Abstract**

A bone-conduction transcranial cerebral pressure monitoring device (100) and a bone-conduction transcranial cerebral pressure monitoring module (10) are provided. The bone-conduction transcranial cerebral pressure monitoring device (100) includes a holding device (1) and a monitoring module. The monitoring module includes at least one accelerometer (2), an output device (3) communicatively connected to the at least one accelerometer (2), and a power supply device (4) that is configured to supply power to the at least one accelerometer (2) and the output device (3). The at least one accelerometer (2) is configured to contact an outer skin of a skull for sensing a transcranial cerebral pressure acceleration wave of a user. The transcranial cerebral pressure acceleration wave is output by the output device (3).

## Description

### FIELD OF THE INVENTION

The present invention relates to a transcranial cerebral pressure monitoring device and a transcranial cerebral pressure monitoring module, and more particularly to a bone-conduction transcranial cerebral pressure monitoring device and a bone-conduction transcranial cerebral pressure monitoring module.

### BACKGROUND OF THE INVENTION

The human skull covers brain tissue, blood, and cerebrospinal fluid. Under normal situations, an internal volume of a cranial cavity remains stable and balanced, and an evenly-distributed positive pressure generated by an intracavitary volume is referred to as an intracranial pressure (ICP). The intracranial pressure is used to evaluate a health status of the damaged brain. This technology is usually adopted in an intensive care unit, especially for patients who have a brain injury, a stroke, a brain tumor, or a neurological surgery.

Use of a conventional invasive ICP monitor is currently the most popular and accurate way of detection. In order to use the invasive ICP monitor, a hole is drilled through the skull of a patient, and a catheter of said monitor is inserted into the brain and connected to an external monitoring device, so as to track and record changes of the intracranial pressure in real time. However, an invasive surgery is accompanied by high costs and risks. In addition, common complications that result from use of the invasive ICP monitor include infection, hemorrhage, blockage, malfunction, and inaccurate positioning. When an intraventricular catheter is implemented for more than one week, chances of infection may increase significantly, and long-term use is not possible.

An existing non-invasive ICP monitor can be implemented by way of, for example, tympanic cavity pressure detection, magnetic induction, phototransduction, and mechanical displacement waveforms. The non-invasive ICP monitor can effectively resolve the above-mentioned problems caused by the invasive surgery. However, compared with the invasive ICP monitor, how the non-invasive ICP monitor performs detection is more indirect, and multi-signal processing (e.g., synthesis, wave filtering, amplification, and conversion) may be required. Hence, a signal that is eventually output is easily distorted, and pathological features are not obvious, such that application in diagnosis is limited. Furthermore, since a signal processor needs to be additionally installed, miniaturization and cost reduction of an entire device cannot be achieved, thereby leaving much room for improvement in the relevant art.

### SUMMARY OF THE INVENTION

In response to the above-referenced technical inadequacies, the present invention provides a bone-conduction transcranial cerebral pressure monitoring device and a bone-conduction transcranial cerebral pressure monitoring module for continuously monitoring physiological data associated with pulsation of the brain. Apart from being low in costs, miniaturized, and simplified in structure, the bone-conduction transcranial cerebral pressure monitoring device and the bone-conduction transcranial cerebral pressure monitoring module provide clear and easy-to-read signals.

In order to resolve the above-mentioned problems, one of the technical aspects adopted by the present invention is to provide a bone-conduction transcranial cerebral pressure monitoring device, which includes a holding device, an accelerometer, an output device, and a power supply device. The accelerometer is disposed on the holding device, and is exposed from a surface of the holding device for contacting an outer skin of a skull of a user, so as to sense a transcranial cerebral pressure acceleration wave of the user for formation of a first monitoring signal. The output device is disposed on the holding device, and is communicatively connected to the accelerometer, so as to output the first monitoring signal. The power supply device is disposed on the holding device, and is electrically connected to the accelerometer and the output device, so as to supply power to the accelerometer and the output device. When the user wears the bone-conduction transcranial cerebral pressure monitoring device, the accelerometer is configured to be in contact with and attached to the outer skin of the skull of the user by the holding device.

In order to resolve the above-mentioned problems, another one of the technical aspects adopted by the present invention is to provide a bone-conduction transcranial cerebral pressure monitoring module, which includes an accelerometer, an output device, and a power supply device. The accelerometer is configured to contact an outer skin of a skull, so as to sense a transcranial cerebral pressure acceleration wave of a user for formation of a first monitoring signal. The output device is communicatively connected to the accelerometer, so as to output the first monitoring signal. The power supply device is electrically connected to the accelerometer and the output device, so as to supply power to the accelerometer and the output device.

Therefore, in the bone-conduction transcranial cerebral pressure monitoring device and the bone-conduction transcranial cerebral pressure monitoring module provided by the present invention, by virtue of "the accelerometer being configured to contact the outer skin of the skull, so as to sense the transcranial cerebral pressure acceleration wave of the user for formation of the first monitoring signal," a new alternative for non-invasive monitoring of physiological data associated with an intracranial pressure can be obtained. Apart from providing a wearable sensor that is low in costs and miniaturized, a transcranial cerebral pressure (TCCP) waveform of high precision can also be provided through a non-invasive manner. The most significant feature of the TCCP waveform is its large output amplitude (unit: volts), such that waveform details can be more noticeable and easily identifiable. Compared with other conventional non-invasive intracranial pressure waveforms, the TCCP waveform is visually distinct, thereby significantly improving capabilities of identifying various physiological conditions associated with the intracranial pressure. In this way, a diagnosis process can be more intuitive, and the accuracy for identifying abnormalities of the intracranial pressure can also be greatly enhanced, such that minor pressure changes or important physiological signals can all be detected and analyzed in a more precise manner.

These and other aspects of the present invention will become apparent from the following description of the embodiment taken in conjunction with the following drawings and their captions, although variations and modifications therein may be affected without departing from the spirit and scope of the novel concepts of the invention.

### BRIEF DESCRIPTION OF THE DRAWINGS

The described embodiments may be better understood by reference to the following description and the accompanying drawings, in which:
FIG. 1 is a functional block diagram of a bone-conduction transcranial cerebral pressure monitoring module according to a first embodiment of the present invention;
FIG. 2 is a schematic diagram showing a bone-conduction transcranial cerebral pressure monitoring device in use according to the first embodiment of the present invention;
FIG. 3A is a schematic perspective view showing an appearance of a main body (a hollow housing) of the bone-conduction transcranial cerebral pressure monitoring device according to the first embodiment of the present invention;
FIG. 3B is another schematic perspective view showing the appearance of the main body (the hollow housing) of the bone-conduction transcranial cerebral pressure monitoring device according to the first embodiment of the present invention;
FIG. 4 is a schematic cross-sectional view showing the appearance of the main body (the hollow housing) of the bone-conduction transcranial cerebral pressure monitoring device according to the first embodiment of the present invention;
FIG. 5 is a schematic cross-sectional view showing an enlarged part of the bone-conduction transcranial cerebral pressure monitoring device according to the first embodiment of the present invention;
FIG. 6 is a flowchart of a bone-conduction transcranial cerebral pressure monitoring method according to the first embodiment of the present invention;
FIG. 7 is a schematic diagram exemplifying a first monitoring signal according to the present invention;
FIG. 8A is a schematic diagram exemplifying the first monitoring signal according to the present invention;
FIG. 8B is a schematic diagram exemplifying the first monitoring signal according to the present invention;
FIG. 9 is a schematic diagram showing transcranial cerebral pressure waveforms when a left side and a right side of a brain of a user are being simultaneously monitored;
FIG. 10 is a functional block diagram of the bone-conduction transcranial cerebral pressure monitoring module according to a second embodiment of the present invention;
FIG. 11 is a schematic diagram exemplifying a second monitoring signal according to the present invention; and
FIG. 12 is a flowchart of the bone-conduction transcranial cerebral pressure monitoring method according to the second embodiment of the present invention.

### DETAILED DESCRIPTION OF THE EXEMPLARY EMBODIMENTS

The present invention is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a," "an" and "the" includes plural reference, and the meaning of "in" includes "in" and "on." Titles or subtitles can be used herein for the convenience of a reader, which shall have no influence on the scope of the present invention.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present invention or of any exemplified term. Likewise, the present invention is not limited to various embodiments given herein. Numbering terms such as "first," "second" or "third" can be used to describe various components, signals or the like, which are for distinguishing one component/signal from another one only, and are not intended to, nor should be construed to impose any substantive limitations on the components, signals or the like.

### [First Embodiment]

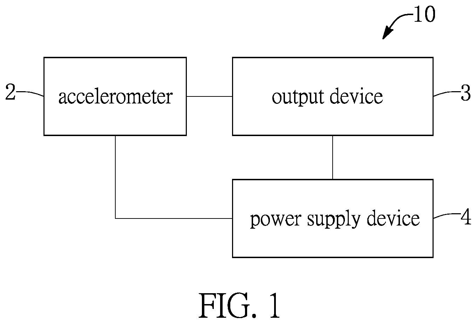

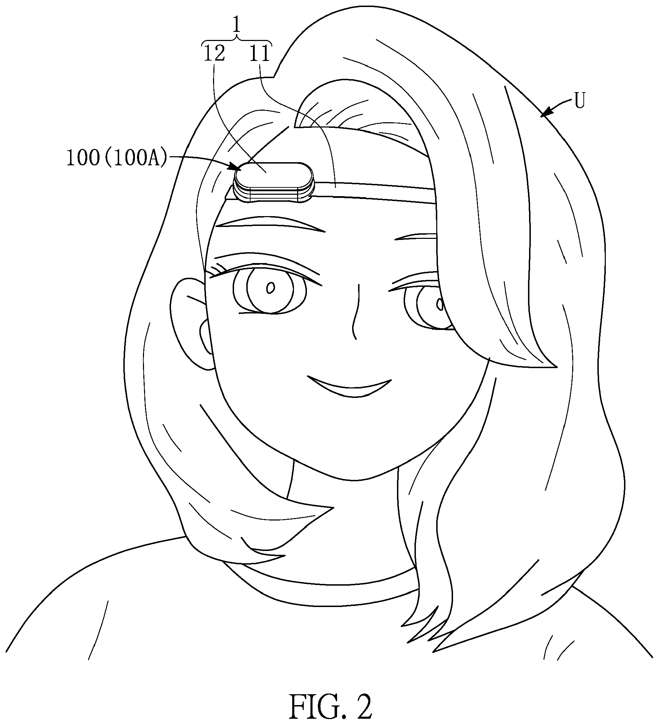

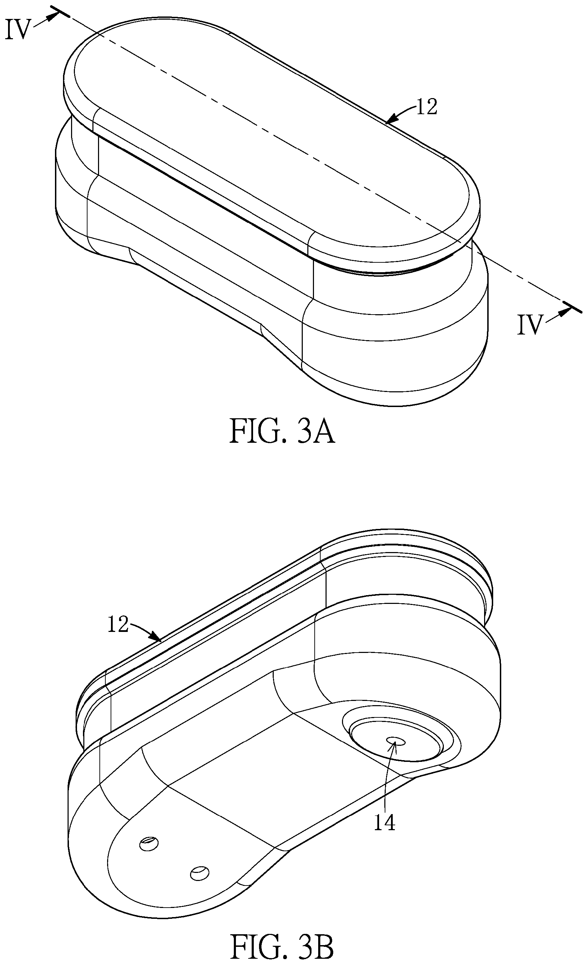

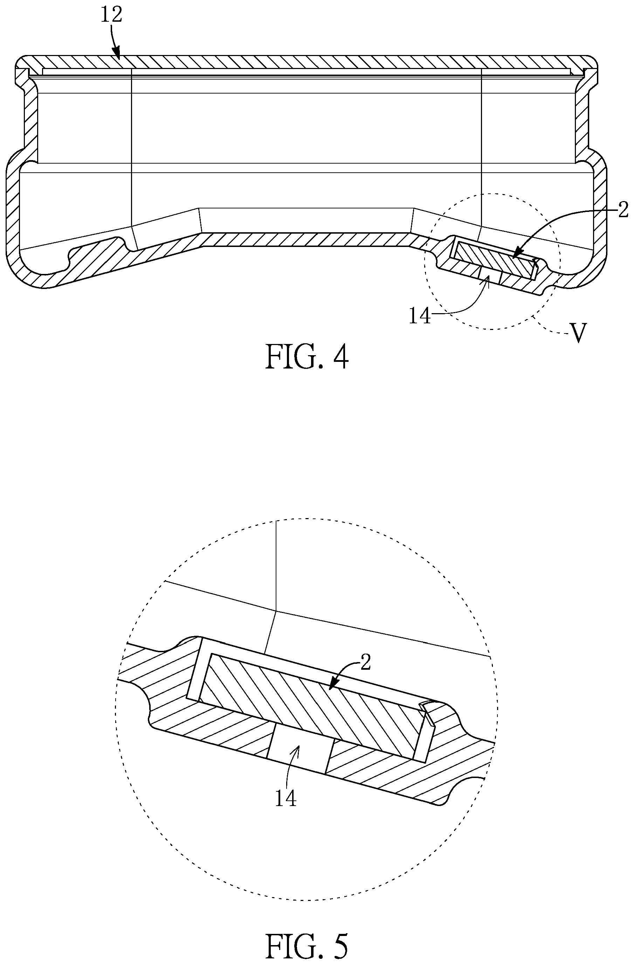

Reference is made to FIG. 1 to FIG. 5. FIG. 1 is a block diagram of a bone-conduction transcranial cerebral pressure monitoring module 10 according to a first embodiment of the present invention, and FIG. 2 is a schematic diagram showing a bone-conduction transcranial cerebral pressure monitoring device 100 in use according to the first embodiment of the present invention. FIG. 3A and FIG. 3B are each a schematic perspective view showing an appearance of a main body 12 of the bone-conduction transcranial cerebral pressure monitoring device 100 according to the first embodiment of the present invention. FIG. 4 is a schematic cross-sectional view showing the appearance of the main body 12 of the bone-conduction transcranial cerebral pressure monitoring device 100 according to the first embodiment of the present invention. FIG. 5 is a schematic cross-sectional view showing an enlarged part of the bone-conduction transcranial cerebral pressure monitoring device 100 according to the first embodiment of the present invention.

As shown in FIG. 1, the bone-conduction transcranial cerebral pressure monitoring module 10 in the first embodiment of the present invention includes at least one accelerometer 2, an output device 3, and a power supply device 4.

The accelerometer 2 is configured to contact an outer skin of a skull of a user, so as to sense a transcranial cerebral pressure acceleration wave (a first monitoring signal L 1) of the user in a bone-conduction manner.

The accelerometer 2 is attached to the outer skin of the skull of the user (a test subject) for continuously sensing the transcranial cerebral pressure acceleration wave, such that a transcranial cerebral pressure (TCCP) is formed. In conventional intracranial pressure (ICP) waveforms, the transcranial cerebral pressure is a Laplacian-of-Gaussian (LoG) type waveform, and is a relatively novel way of evaluating brain dynamics (details thereof will be illustrated below).

The output device 3 is configured to output a sensing result of the accelerometer 2 for an interpreter to access or identify.

In the present embodiment, the output device 3 is a wireless transmission module, and can transmit the sensing result of the accelerometer 2 to other receiving devices in a wireless manner. For example, the sensing result (e.g., the first monitoring signal L1 or other monitoring signals) is transmitted to a printer, a computer, a tablet computer, a smartphone, a medical display device, etc. That is, the output device 3 can transmit the sensing result of the accelerometer 2 to the interpreter's smartphone, computer, or display screen or speaker (having a wireless receiving function) for their use. The wireless transmission module can adopt any structure or transmission method that may achieve the above-mentioned purpose, such as BLUETOOTH, ZigBee, WI-FI, or any other remote communication method. Since how the wireless transmission module is implemented is not the focus of the present invention, details thereof will not be elaborated herein. In addition, the output device 3 can also be a wired transmission module or a display device or speaker disposed on a holding device 1.

The power supply device 4 can be a wired power source, a wireless power source, a battery, or other existing power supply devices. Since how the power supply device is implemented is not the focus of the present invention, details thereof will not be elaborated herein.

Reference is made to FIG. 3A, FIG. 3B, FIG. 4, and FIG. 5. The bone-conduction transcranial cerebral pressure monitoring device 100 in the first embodiment of the present invention includes the holding device 1, the at least one accelerometer 2, the output device 3, and the power supply device 4. In other words, the bone-conduction transcranial cerebral pressure monitoring device 100 includes the bone-conduction transcranial cerebral pressure monitoring module 10 and the holding device 1. The bone-conduction transcranial cerebral pressure monitoring device 100 is fixed to the head of a user U, and the accelerometer 2 is in contact with and attached to an outer skin of a skull of the user U. Accordingly, physiological data associated with a transcranial cerebral pressure of the user U can be monitored in a non-invasive manner. Here, the accelerometer 2, the output device 3, and the power supply device 4 are disposed on the holding device 1.

Referring to FIG. 2, the accelerometer 2 is disposed on the head of the user U by being attached to and in contact with the outer skin of the skull of the user U via the holding device 1.

In the present embodiment, the holding device 1 includes a headband 11 and the main body 12. The headband 11 is used for fixing the main body 12 to the head of the user U, and can be a non-elastic strap, an adjustable strap, or an elastic strap (but is not limited thereto). While the main body 12 is fixed to the head of the user U by the headband 11 in the present embodiment, the present invention is not limited thereto. In other embodiments (not shown in the figures), the main body 12 can also be fixed to the head of the user U by use of a head cover or ear hooks, by adhesion, or even by manual pressing.

In the present embodiment, in addition to having the function of fixing the main body 12 to the head of the user U, the headband 11 can also be used as a biasing device by applying to the main body 12 a bias directed toward the head of the user U. The biasing device enables the accelerometer 2 disposed on the main body 12 to be more adjacent to the skull of the user U, so as to improve the intensity and the clearness of a bone-conduction signal. However, the biasing device is not limited to being the headband 11, and can take any form (e.g., a shrinkable adhesive) as long as it is capable of applying to the main body 12 (the accelerometer 2) the bias directed toward the head of the user U.

Referring to FIG. 3A and FIG. 3B, the main body 12 of the present embodiment is a hollow housing, and has a through hole 14. The through hole 14 allows the accelerometer 2 to be disposed in the main body 12 and be exposed from a surface of the main body 12. However, the present invention is not limited thereto. In other embodiments (not shown in the figures), the main body 12 can be, for example, a substrate or a patch, and the accelerometer 2 can be configured to be flush with or even protrude from the surface of the main body 12. Any configuration that is capable of carrying the accelerometer 2, the output device 3, and the power supply device 4 and allows the accelerometer 2 to be exposed from the surface of the main body 12 is allowable.

Through the holding device 1, the accelerometer 2 can be disposed on the head of the user U by being attached to and in contact with the outer skin of the skull of the user U, so as to continuously monitor a transcranial cerebral pressure acceleration wave of the user U.

The output device 3 is disposed on the holding device 1, and is communicatively connected to the accelerometer 2. The power supply device 4 is disposed on the holding device 1, and is connected to supply power to the accelerometer 2 and the output device 3.

It should be noted that the focus of the present invention is the bone-conduction transcranial cerebral pressure monitoring module 10 that includes the accelerometer 2, the output device 3, and the power supply device 4. In other words, how the holding device 1 is implemented is not the focus of the present invention. As long as the accelerometer 2 can be in contact with and attached to the outer skin of the skull of the user U, and the output device 3 outputs the first monitoring signal L1, the specific technical effect of the present invention can be achieved.

The accelerometer 2 can be a capacitive accelerometer, a piezoelectric accelerometer, a piezoresistive accelerometer, an optical accelerometer, or an MEMS (micro-electromechanical systems) accelerometer. The capacitive accelerometer has high sensitivity and low power consumption, and is suitable for applications where long-term stability is needed. The piezoelectric accelerometer has a wide dynamic range and a high frequency response, and is often used in an environment of high vibration. The piezoresistive accelerometer has a simple structure and is low in manufacturing costs, but may be affected by temperature changes. The optical accelerometer has advantages of having high precision and not being easily affected by interferences, but is high in costs. The MEMS accelerometer is widely applied to consumer electronics due to its miniaturization, low power consumption, and low costs. Since the accelerometer 2 senses the physiological data associated with the transcranial cerebral pressure through bone conduction, the accelerometer 2 is preferably the capacitive accelerometer or the MEMS accelerometer.

Specifically, the capacitive accelerometer has advantages of having high sensitivity and stability. When being installed in a wearable device for detecting an acceleration wave that is bone-conducted (conduction in solids), the capacitive accelerometer can precisely detect minute changes in acceleration. In addition, the capacitive accelerometer usually has good long-term stability (which is important for a wearable device that is to be worn for a long period of time), and its low power consumption is beneficial for prolonging a battery service life of a device.

On the other hand, the MEMS accelerometer has advantages of miniaturization, low costs, and low power consumption when being used in the wearable device. The MEMS accelerometer has a small volume, and is very suitable for a wearable device that is limited in volume. In terms of manufacturing cost-effectiveness, the MEMS accelerometer is an ideal choice for mass production. The characteristic of low power consumption ensures feasibility of long-term operation, and the MEMS accelerometer can also be easily integrated with other electronic components, thereby allowing the wearable device to achieve multi-functionality.

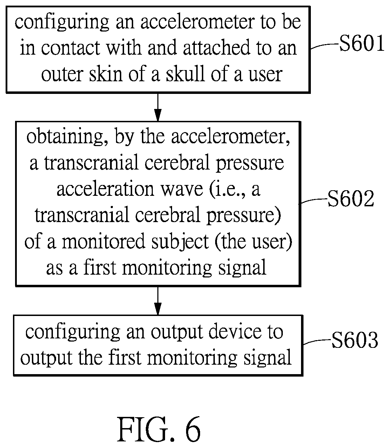

Referring to FIG. 6, a bone-conduction transcranial cerebral pressure monitoring method that is achieved by using the bone-conduction transcranial cerebral pressure monitoring device 100 or the bone-conduction transcranial cerebral pressure monitoring module 10 of the present embodiment is further illustrated.

Step S601: configuring the accelerometer 2 of the bone-conduction transcranial cerebral pressure monitoring device 100 or the bone-conduction transcranial cerebral pressure monitoring module 10 (at the through hole 14 of the main body 12 in the present embodiment) to be in contact with and attached to the outer skin of the skull of the user U.

Step S602: obtaining, by the accelerometer 2, the transcranial cerebral pressure acceleration wave (i.e., the transcranial cerebral pressure) of a monitored subject (the user U) as the first monitoring signal L1.

Step S603: configuring the output device 3 to output the first monitoring signal L1.

Through the above-mentioned bone-conduction transcranial cerebral pressure monitoring method, the transcranial cerebral pressure for monitoring the user U can be obtained.

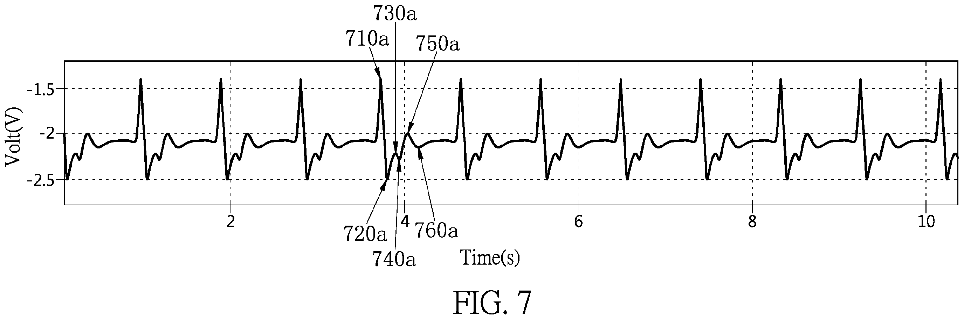

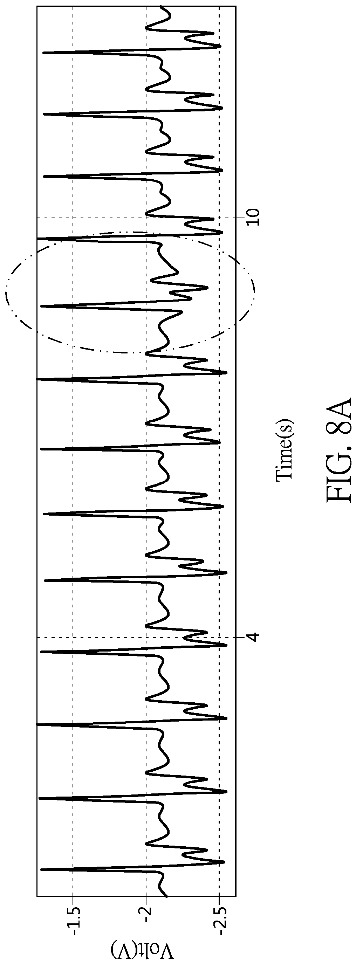

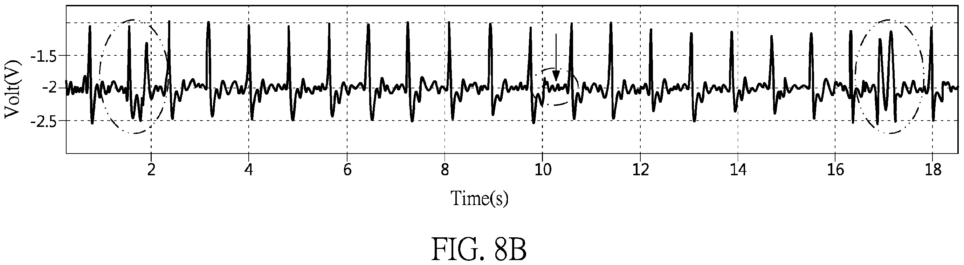

Referring to FIG. 7, FIG. 8A, and FIG. 8B, the transcranial cerebral pressure (the first monitoring signal L1) obtained by the bone-conduction transcranial cerebral pressure monitoring method of the present embodiment and its application in relevant physiological conditions will be illustrated in the following descriptions.

The transcranial cerebral pressure (TCCP) is a relatively novel way of evaluating brain dynamics. As shown in FIG. 7, the transcranial cerebral pressure is a continuous waveform, and each cycle has six inflection points (i.e., a first inflection point 710a, a second inflection point 720a, a third inflection point 730a, a fourth inflection point 740a, a fifth inflection point 750a, and a sixth inflection point 760a). In measurement of the transcranial cerebral pressure, the observed continuous waveform exhibits an amplitude on the order of volts (V). This feature is distinguishable from signals on the order of microvolts (µY) or millivolts (mV), and an intensity of a TCCP signal is clearly in a higher voltage class. By observing the change of each inflection point in each cycle of the transcranial cerebral pressure, the interpreter can more easily observe the physiological data associated with the transcranial cerebral pressure of the monitored subject (the user U). In this way, pathological problems that the monitored subject may have can be more accurately determined. It should be noted that the interpreter mentioned herein refers to a person that has experience or knowledge in the intracranial pressure waveforms and diagnosis and is capable of making pathological determinations according to the physiological data associated with the transcranial cerebral pressure.

As mentioned above, the transcranial cerebral pressure (TCCP) can be used to monitor minute changes of an intracranial pressure, so as to reflect possible abnormalities of the intracranial pressure. Specifically, according to the Monro-Kellie doctrine, an overall volume of intracranial fluid changes in response to an increase or a decrease of the intracranial pressure, and a cerebral perfusion pressure (CPP) is calculated by subtracting the intracranial pressure (ICP) from a mean arterial pressure (MAP), the formula thereof being: CPP = MAP - ICP. While relative constancy of the intracranial pressure is crucial to maintenance of the cerebral perfusion pressure, the cerebral perfusion pressure determines an overall cerebral blood flow (CBF). If a certain portion of the skull is increased in volume, other portions need to be reduced in volume for maintaining the balance and a normal intracranial pressure. The cerebral perfusion pressure is a pressure of the blood flowing to the brain, and is usually constant due to autoregulation. Regarding an abnormal mean arterial pressure or an abnormal intracranial pressure, the main danger of an increase of the intracranial pressure is a decrease of the cerebral perfusion pressure due to the autoregulation mechanism of the body, thereby resulting in a decrease of the overall cerebral blood flow and ischemia. In other words, if the intracranial pressure is abnormal and is close to the level of the mean arterial pressure, the cerebral perfusion pressure will be decreased. In response to a decreased cerebral perfusion pressure, the blood pressure of the whole body is increased, and cerebral vessels are expanded. As a result, a cerebral blood volume is increased once again, such that a vicious cycle is formed as the intracranial pressure is increased and the cerebral perfusion pressure is further decreased. This may lead to a significant decrease of the cerebral blood volume and the cerebral perfusion pressure, thereby causing ischemia and cerebral infarction. Such an abnormal increase and an abnormal decrease of the cerebral perfusion pressure can be reflected in real-time pressure pulsation of the brain (i.e., the transcranial cerebral pressure). A cerebral pulse pressure formed by pressure surges of the brain is continuously monitored, and the transcranial cerebral pressure can be used to evaluate a health status of the damaged brain. The focus of monitoring transcranial cerebral pressure surges is to measure and analyze pressure changes of cerebrospinal fluid, which is beneficial to identifying potential dangers (such as cerebral edema, intracerebral hemorrhage, or intracranial hypertension).

Regarding practical applications of the transcranial cerebral pressure formed in the present embodiment, FIG. 8A and FIG. 8B are provided for illustrating examples in which the transcranial cerebral pressure is used to determine various possible pathological problems.

In the example of FIG. 8A, the monitored subject is a young male with a history of taking an anxiolytic, and claims to be in a temporary panicked state during the monitoring process. At a time point that corresponds to the panicked state during the monitoring process, it can be observed that the second inflection point 720a in the cycle of the transcranial cerebral pressure is significantly decreased.

In the example of FIG. 8B, the monitored subject has been troubled by sleep disorder for a long time, and has no history of heart disease. After diagnosis, right carotid artery stenosis is discovered, and obvious occurrences of pressure turbulence (numerous circled parts) can be observed from a transcranial cerebral pressure (TCCP) waveform of the monitored subject.

The examples above show that the transcranial cerebral pressure monitoring technique can be effectively applied to identification and evaluation of various brain issues and relevant pathological problems. In the example of FIG. 8A, when the young male being monitored is in the panicked state, the TCCP waveform clearly reflects such psychological change. This indicates that the transcranial cerebral pressure monitoring technique has the potential in perceiving and recording physiological reactions caused by emotions. In the example of FIG. 8B, for the monitored subject who has been troubled by the sleep disorder for a long time and is diagnosed with the carotid artery stenosis, the TCCP waveform exhibits obvious occurrences of pressure turbulence. This further demonstrates the effectiveness of the transcranial cerebral pressure monitoring technique in monitoring more complex pathological conditions of the brain. Accordingly, the transcranial cerebral pressure monitoring technique can not only provide insights into physiological conditions associated with the intracranial pressure, but can also act as an important tool for diagnosing and monitoring the health status of the brain.

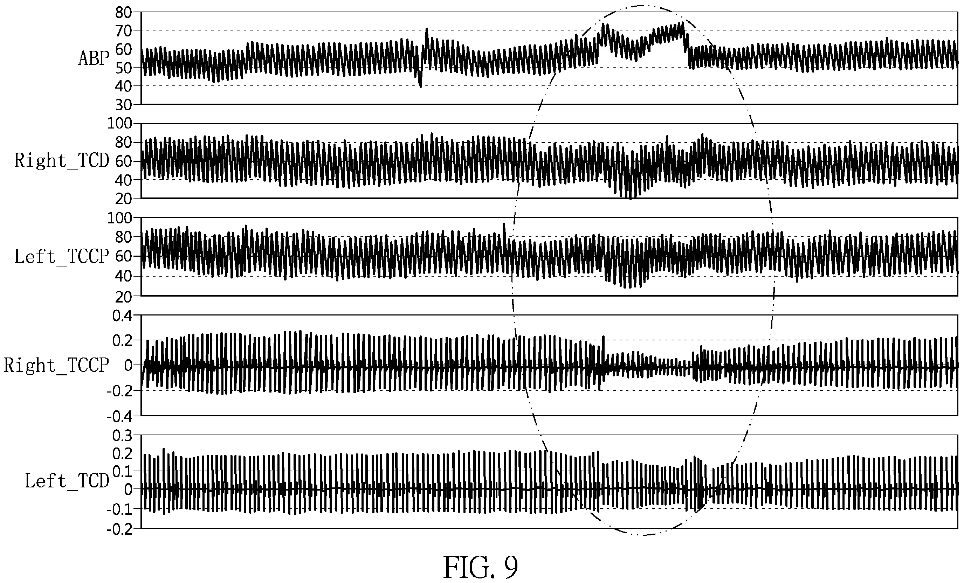

FIG. 9 shows application of data obtained by simultaneously monitoring the transcranial cerebral pressure of a left side and a right side of the brain of the user U. In one embodiment (not shown in the figures), in order to sense the transcranial cerebral pressure of the left side and the right side of the brain (as shown in FIG. 9), a quantity of the accelerometer 2 is two or more, and the two or more accelerometers 2 are disposed on the holding device 1 and spaced apart from each other by a predetermined distance. In this way, when the user U wears the holding device 1, at least one of the two or more accelerometers 2 is in contact with and attached to the outer skin of the left side of the skull of the user U, and at least another one of the two or more accelerometers 2 is in contact with and attached to the outer skin of the right side of the skull of the user U, so as to simultaneously monitor the transcranial cerebral pressure of the left side and the right side of the brain of the user U.

The waveform of a left-side brain monitoring signal (left TCCP) and the waveform of a right-side brain monitoring signal (right TCCP) obtained by the two accelerometers 2 are shown in FIG. 9. In FIG. 9, the monitored subject is a young female with left vena stenosis, and real-time changes of physiological data associated with the intracranial pressure are provided by observing the transcranial cerebral pressure that reflects pathological conditions of the left vena stenosis. Waveform vibrations indicate abnormalities (an area within the dotted oval), and insufficiency in autoregulation (i.e., the transcranial cerebral pressure of the right side of the brain is greater than an insufficiency of the cerebral perfusion pressure of the left side of the brain) due to a decrease of the cerebral perfusion pressure of the right side of the brain being greater than that of the left side of the brain can be observed therefrom.

For the left-side brain monitoring signal (left TCCP) and the right-side brain monitoring signal (right TCCP), the abnormalities can be clearly identified merely by comparing the waveforms of the left side and the right side. As for waveforms (such as transcranial Doppler (TCD) or an arterial blood pressure (ABP)) sensed by other non-invasive testing devices (as shown in FIG. 9), there are no obvious differences between the waveforms of the left side and the right side of the brain.

Based on the above, from the physiological data associated with the transcranial cerebral pressure, the interpreter (a diagnoser) can obtain more useful information for having a better understanding of the pathological conditions of the monitored subject (the user U).

The advantages of using the transcranial cerebral pressure for monitoring the physiological data associated therewith are as follows.

One advantage is that placement of an invasive catheter during an evaluation process is not required. Measurement of the transcranial cerebral pressure is non-invasive. By attaching the accelerometer 2 to (the outer skin of) the skull (e.g., the frontal bone), the Laplacian-of-Gaussian (LoG) type waveform (i.e., the transcranial cerebral pressure waveform) can be obtained in a bone-conduction manner.

Another advantage is an excellent signal-to-noise ratio. Due to having a good signal-to-noise ratio, the transcranial cerebral pressure can provide clearer and more accurate brain dynamics, and is more resistant to interferences and noise. Such features are crucial to accurately evaluating a circulation status and stably monitoring the cerebral blood flow and dynamic changes of the pressure.

Yet another advantage is the high frequency response ability. In the technology of the transcranial cerebral pressure, high frequency components in the cerebral pressure are detectable. These high frequency components are crucial to evaluating rapid and dynamic changes of the blood in the brain.

Still another advantage is miniaturization of the device and 24/7 monitoring of cerebral pressure (i.e., continuous services or monitoring for 24 hours a day, seven days a week).

In the present embodiment, through bone conduction, real-time parameters for intracranial blood dynamics can be obtained simply by attaching the accelerometer 2 to the outer skin of the skull, and these real-time parameters are provided for pathological interpretation or reference. The non-invasive transcranial cerebral pressure monitoring in real time is also beneficial to identifying and quantifying abnormal changes. Furthermore, apart from the accelerometer 2, no other processor (for wave filtering or signal amplification and conversion) is needed. In the application of telehealth, popularization of a monitoring system (even in outlying islands or remote areas) can be promoted due to overall miniaturization, portability, and easy operation. Accordingly, the transcranial cerebral pressure can be used as a non-invasive tool for monitoring the cerebral blood flow, so as to enhance medical care and improve treatment effects.

### [Second Embodiment]

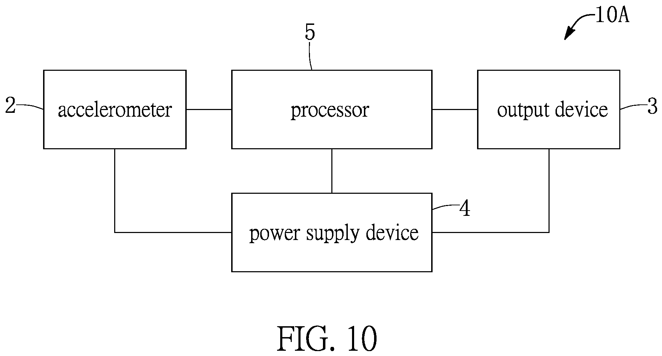

Referring to FIG. 10, a second embodiment of the present invention provides a bone-conduction transcranial cerebral pressure monitoring module 10A. Apart from adding a processor 5 (an integral calculator), the bone-conduction transcranial cerebral pressure monitoring module 10A in the second embodiment of the present invention is substantially the same as that of the first embodiment, and the similarities therebetween will not be reiterated herein.

In the present embodiment, the processor 5 is disposed on the holding device 1, and is communicatively connected to the accelerometer 2 and the output device 3. The power supply device 4 is configured to supply power to the accelerometer 2, the output device 3, and the processor 5. The processor 5 is configured to perform a double integral on the first monitoring signal L1 output by the accelerometer 2, so as to form a second monitoring signal L2. The output device 3 outputs the second monitoring signal L2.

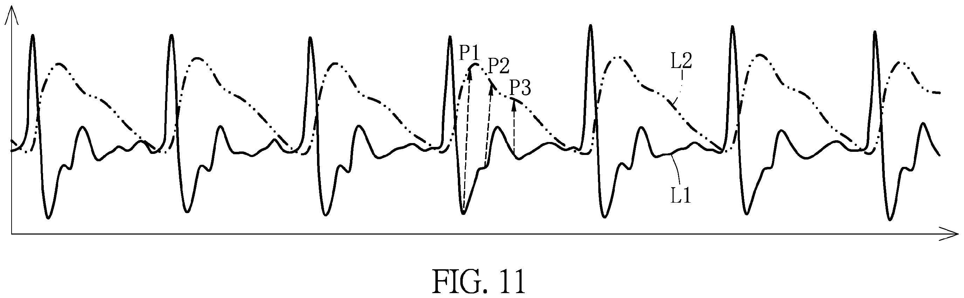

As shown in FIG. 11, the second monitoring signal L2 is a perfusion pressure waveform of the transcranial cerebral pressure. This waveform is similar to the intracranial pressure (ICP) waveform associated with an individual and heartbeats, and has three determination values (i.e., P1, P2, and P3) that are provided to the interpreter for observation and interpretation of the pressure waveform. The ICP waveform associated with the individual and the heartbeats is a graph that reflects changes of the intracranial pressure during beating of the heart. Here, P1, P2, and P3 are the three main peaks, each of which represents a different physiological condition. P1 (a percussion wave) is the first peak in the waveform, and is usually the highest. P1 represents influences of an arterial blood flow on brain tissue and the cerebrospinal fluid (CSF), and reflects a real-time pressure effect of a cardiac output on the cerebral vessels. P2 (a rebound wave) is the second peak in the waveform. Compared to P1, a height of P2 is likely to change according to elasticity of the brain tissue and a status of cerebrospinal fluid dynamics, and is usually associated with cerebral flexibility and a reaction of the brain tissue to an initial increase of the blood flow. P3 (a dicrotic wave) is the third peak in the waveform, and is usually the lowest. This peak is associated with the arterial blood pressure and rebound of a mitral valve, and is also associated with a latter part of a cardiac cycle, thereby reflecting blood flow dynamics of a latter period of the heartbeat. Due to providing information of cerebral blood flow dynamics, the elasticity of the brain tissue, and a pressure status of the cerebrospinal fluid, these peaks can be used to evaluate a status of the intracranial pressure.

An invasive method for obtaining the ICP waveform can provide the most direct and most accurate measurement, but involve high risks (which include infection and hemorrhage). A non-invasive method has lower risks, but often provides an indirect or speculative measurement result. In the conventional technology, the measured waveform can hardly reach a raw measurement signal (i.e., the amplitude on the order of volts (V)). Since subsequent signal processing (e.g., amplification and wave filtering) cannot be omitted, serious signal distortion during interpretation is likely to occur. In comparison, an ICP waveform monitoring method provided in the present embodiment allows a waveform similar to the ICP waveform to be obtained with less computation costs and a greater amplitude, and is a new alternative for obtaining the ICP waveform.

It should be noted that, in the present embodiment, the output device 3 is configured to output the second monitoring signal L2. However, in cooperation with the interpretation requirements of the interpreter, the output device 3 can also output the first monitoring signal L1, or output the first monitoring signal L1 and the second monitoring signal L2.

Moreover, different from the bone-conduction transcranial cerebral pressure monitoring device 100 of the first embodiment, a bone-conduction transcranial cerebral pressure monitoring device 100A further includes the processor 5. The bone-conduction transcranial cerebral pressure monitoring device 100A is disposed on the head of the user U, and continuously outputs the second monitoring signal L2, such that the interpreter may continuously monitor the physiological data associated with the transcranial cerebral pressure of the user U.

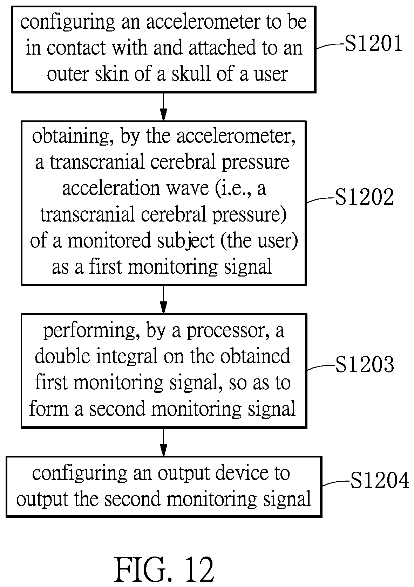

Referring to FIG. 12, a bone-conduction transcranial cerebral pressure monitoring method that is achieved by using the bone-conduction transcranial cerebral pressure monitoring device 100A or the bone-conduction transcranial cerebral pressure monitoring module 10A of the second embodiment is further illustrated.

Step S1201: configuring the accelerometer 2 of the bone-conduction transcranial cerebral pressure monitoring device 100A (at the through hole 14 of the main body 12 in the present embodiment) to be in contact with and attached to the outer skin of the skull of the monitored subject (the user U).

Step S1202: obtaining, by the accelerometer 2, the transcranial cerebral pressure acceleration wave (i.e., the transcranial cerebral pressure) as the first monitoring signal L1.

Step S1203: performing, by the processor 5, a double integral on the first monitoring signal L1 obtained in the above-mentioned step S 1102, so as to form the second monitoring signal L2.

Step S1024: configuring the output device 3 to output the second monitoring signal L2.

Through the above-mentioned bone-conduction transcranial cerebral pressure monitoring method, the perfusion pressure waveform of the transcranial cerebral pressure (i.e., the ICP waveform associated with the individual and the heartbeats) for monitoring the user U can be obtained.

### [Beneficial Effects of the Embodiments]

In conclusion, the accelerometer 2 used in the bone-conduction transcranial cerebral pressure monitoring device 100 of the present invention can monitor the physiological data associated with the transcranial cerebral pressure of the user in a non-invasive manner. In other words, the bone-conduction transcranial cerebral pressure monitoring device and the bone-conduction transcranial cerebral pressure monitoring module of the present invention are new alternatives for the user to non-invasively monitor the physiological data associated with the intracranial pressure. Furthermore, the bone-conduction transcranial cerebral pressure monitoring device of the present invention is a wearable sensor that is low in costs and miniaturized, and is capable of providing a waveform of the physiological data associated with the intracranial pressure (the TCCP waveform) in a non-invasive manner. Compared with other existing waveforms for non-invasive monitoring of the physiological data associated with the intracranial pressure, said waveform provides clear and easy-to-read signals, such that different physiological conditions associated with the intracranial pressure can be clearly reflected.

The foregoing description of the exemplary embodiments of the invention has been presented only for the purposes of illustration and description and is not intended to be exhaustive or to limit the invention to the precise forms disclosed. Many modifications and variations are possible in light of the above teaching.

The embodiments were chosen and described in order to explain the principles of the invention and their practical application so as to enable others skilled in the art to utilize the invention and various embodiments and with various modifications as are suited to the particular use contemplated. Alternative embodiments will become apparent to those skilled in the art to which the present invention pertains without departing from its spirit and scope.

## Claims

1. A bone-conduction transcranial cerebral pressure monitoring device (100), **characterized by** comprising:
a holding device (1);
at least one accelerometer (2), wherein the at least one accelerometer (2) is disposed on the holding device (1), and is exposed from a surface of the holding device (1) for contacting an outer skin of a skull of a user, so as to sense a transcranial cerebral pressure acceleration wave of the user for formation of a first monitoring signal (L1);
an output device (3), wherein the output device (3) is disposed on the holding device (1), and is communicatively connected to the at least one accelerometer (2), so as to output the first monitoring signal (L1); and
a power supply device (4), wherein the power supply device (4) is disposed on the holding device (1), and is electrically connected to the at least one accelerometer (2) and the output device (3), so as to supply power to the at least one accelerometer (2) and the output device (3);
wherein, when the user wears the bone-conduction transcranial cerebral pressure monitoring device (100), the at least one accelerometer (2) is configured to be in contact with and attached to the outer skin of the skull of the user by the holding device (1).

2. The bone-conduction transcranial cerebral pressure monitoring device (100) according to claim 1, wherein the holding device (1) further includes a hollow housing, and the at least one accelerometer (2), the output device (3), and the power supply device (4) are disposed in the hollow housing.

3. The bone-conduction transcranial cerebral pressure monitoring device (100) according to claim 1, wherein the holding device (1) further includes a biasing structure; wherein, when the at least one accelerometer (2) is attached to the outer skin of the skull of the user by the holding device (1), a bias directed toward the user is applied to the at least one accelerometer (2) by the biasing structure.

4. The bone-conduction transcranial cerebral pressure monitoring device (100) according to claim 1, further comprising a processor (5), wherein the processor (5) is disposed on the holding device (1), and is configured to perform a double integral on the first monitoring signal (L1) output by the at least one accelerometer (2), so as to form a second monitoring signal (L2); wherein the output device (3) outputs at least one of the second monitoring signal (L2) or the first monitoring signal (L 1).

5. The bone-conduction transcranial cerebral pressure monitoring device (100) according to claim 1, wherein the output device (3) is a signal transmission part, and includes at least one of a wired transmission module or a wireless transmission module, so as to transmit the first monitoring signal (L1) to an external device.

6. The bone-conduction transcranial cerebral pressure monitoring device (100) according to claim 1, wherein the output device (3) is at least one of a display device or a speaker.

7. The bone-conduction transcranial cerebral pressure monitoring device (100) according to claim 1, wherein a quantity of the at least one accelerometer (2) is two or more; wherein the accelerometers (2) are disposed on the holding device (1) and spaced apart from each other by a predetermined distance, such that the accelerometers (2) are simultaneously and respectively in contact with and attached to the outer skin of a left side of the skull and the outer skin of a right side of the skull when the user wears the bone-conduction transcranial cerebral pressure monitoring device (100).

8. The bone-conduction transcranial cerebral pressure monitoring device (100) according to claim 1, wherein the at least one accelerometer (2) is a capacitive accelerometer, a piezoelectric accelerometer, or a piezoresistive accelerometer.

9. A bone-conduction transcranial cerebral pressure monitoring module (10), **characterized by** comprising:
at least one accelerometer (2), wherein the at least one accelerometer (2) is configured to contact an outer skin of a skull, so as to sense a transcranial cerebral pressure acceleration wave of a user for formation of a first monitoring signal (L 1);
an output device (3), wherein the output device (3) is communicatively connected to the at least one accelerometer (2), so as to output the first monitoring signal (L 1); and
a power supply device (4), wherein the power supply device (4) is electrically connected to the at least one accelerometer (2) and the output device (3), so as to supply power to the at least one accelerometer (2) and the output device (3).

10. The bone-conduction transcranial cerebral pressure monitoring module (10) according to claim 9, further comprising a processor (5), wherein the processor (5) is configured to perform a double integral on the first monitoring signal (L1) output by the at least one accelerometer (2), so as to form a second monitoring signal (L2); wherein the output device (3) outputs at least one of the second monitoring signal (L2) or the first monitoring signal (L1).
